# EUROPÄISCHE PATENTANMELDUNG

(11) **EP 1 092 404 A1**
(43) Veröffentlichungstag der Anmeldung: **18.04.2001**
(21) Anmeldenummer: 99120269.8
(22) Anmeldetag: 11.10.1999
(51) Int. Cl.: A61F 13/00, A61F 15/00, A61F 13/02, A61M 27/00

(54) **Wundabdeckung**

(71) Anmelder: Eurovita AG, 9490 Vaduz (LI)
(72) Erfinder: Behere, Hans-Jürgen, D-79294 Sölden (DE)
(74) Vertreter: Urner, Peter, Dipl.-Phys.

(57) **Zusammenfassung**

Die Erfindung betrifft eine Wundabdeckung mit einem ringförmigen Befestigungsabschnitt (10), der einen offenen Bereich (11) umschließt, und mit einer den offenen Bereich (11) überdeckenden Haube (13), die mit ihrem Umfangsabschnitt (14) luftdicht mit dem Befestigungsabschnitt (10) verbunden ist und deren über dem offenen Bereich (11) liegender Deckenabschnitt (15) einen Abstand zu einer durch den Befestigungsabschnitt (10) festgelegten Fläche aufweist. Um den Raum (19) über der Wunde möglichst steril halten zu können, ist erfindungsgemäß vorgesehen, daß sich durch die Haube (13) ein absperrbarer Gaseinlaß (17) zum Befüllen der Haube (13) mit einem Gas sowie ein absperrbarer Gasauslaß (18) zum Ablassen des Gases hindurch erstrecken.

## Beschreibung

Die Erfindung betrifft eine Wundabdeckung, wie sie im Oberbegriff des Anspruchs 1 beschrieben ist.

Aus der US 5 817 145 ist bereits eine Wundabdeckung mit einem ringförmigen Befestigungsabschnitt bekannt, der einen offenen Bereich umschließt. An dem Befestigungsabschnitt ist eine Haube angebracht, die in ihrem über dem offenen Bereich liegenden Deckenabschnitt eine Aufnahme für ein Heizelement aufweist, mit dem ein von der Haube umschlossener Behandlungsraum für eine mit dieser bekannten Wundabdeckung abgedeckten Wunde beheizbar ist.

Ferner ist bereits aus der WO 94/00090 eine weitere Wundabdeckung bekannt, die einen mit einem Kleber auf der Haut eines Patienten festlegbaren Dichtring aufweist. Auf diesen Dichtring ist eine ringförmige Luftzuführvorrichtung aufgesetzt, die eine mit Löchern durchbrochene Trennwand zum Überdecken eines Behandlungsraums und ein parallel dazu mit Abstand angeordnetes Filter zum Begrenzen einer über dem Behandlungsraum liegenden Isolationskammer besitzt.

Bei dieser bekannten Wundabdeckung kann warme oder kalte Luft in den Behandlungsraum eingeleitet werden. Aus dem Behandlungsraum über einer Wunde entweicht die zugeführte Luft dann durch die Löcher in der Trennwand in die Isolationskammer, aus der sie durch das Filter austritt.

An Stelle von Luft kann auch ein Sauerstoff und/oder Stickstoffoxid und/oder Wasserdampf enthaltendes Gasgemisch zugeführt werden.

Ferner ist es bereits bekannt, eine luftdichte Wundabdeckung relativ steif auszubilden und mit einem Absauganschluß zu versehen, um auf eine damit abgedeckte Wunde nach dem Evakuieren des Innenraums zwischen Wundabdeckung und Wunde einen Saugeffekt auf das Gewebe im Bereich der Wunde auszuüben, der ähnlich wie beim Schröpfen zur Verbesserung der Durchblutung und zur Beschleunigung der Heilung Blut in den Wundbereich saugt und dabei gleichzeitig das darunter liegende Gewebe mit anhebt.

Der Erfindung liegt die Aufgabe zugrunde, eine Wundabdeckung bereit zu stellen, die es ohne hohen Gasverbrauch ermöglicht, eine Wunde in einer sterilen Umgebung zu halten.

Diese Aufgabe wird durch die Wundabdeckung nach Anspruch 1 gelöst. Vorteilhafte Ausgestaltungen und Weiterbildungen der Erfindung sind in den Unteransprüchen beschrieben. Besonders vorteilhafte Verfahren zur Handhabung der erfindungsgemäßen Wundabdeckung sind in den Verfahrensansprüchen angegeben.

Erfindungsgemäß ist also bei einer Wundabdeckung mit einem Befestigungsabschnitt und einer Haube vorgesehen, daß sich durch die Haube ein absperrbarer Gaseinlaß zum Befüllen der Haube mit einem Gas sowie ein absperrbarer Gasauslaß zum Ablassen des Gases hindurch erstrecken.

Mit dieser Ausbildung der Wundabdeckung wird es ermöglicht, den von der Wundabdeckung über der Wunde gebildeteten Behandlungsraum einerseits mit einem sterilen Gas zu spülen, wobei Gaseinlaß und Gasauslaß offen sind, um nach Beendigung der Spülung des Behandlungsraumes mit sterilem Gas zunächst den Gasauslaß abzusperren, den Behandlungsraum mit Gas zu befüllen, und anschließend nach dem vollständigen Befüllen des Behandlungsraumes mit dem Gas auch den Gaseinlaß abzusperren. Hierbei ist es insbesondere auch möglich, beim Befüllen der Haube einen gewissen Überdruck im Behandlungsraum zu erzeugen, mit dessen Hilfe geprüft werden kann, ob die Wundabdeckung ordnungsgemäß so angebracht ist, daß ein Eindringen von Keimen verhindert wird.

Dabei ist es besonders vorteilhaft, wenn der Gaseinlaß von einer Gasleitung gebildet wird, die sich durch den Umfangsabschnitt der Haube gasdicht hindurch erstreckt, in der ein Absperrventil angeordnet ist und an die in Strömungsrichtung vor dem Absperrventil eine Gasquelle anschließbar ist, wobei das Absperrventil von einem Rückschlagventil gebildet wird.

Um eine kontrollierte Behandlung der Wunde zu ermöglichen und gleichzeitig den Gasverbrauch möglichst gering zu halten, ist es besonders zweckmäßig, wenn die Gasquelle einen Gasvorratsbehälter und eine als Dosiereinrichtung dienende Blasebalgpumpe umfaßt. Dabei ist es vorteilhaft, wenn die Gasquelle als Gasvorratsbehälter einen Druckbehälter aufweist, der mit einem unter Druck stehenden sterilen, keimtötenden und/oder das Keimwachstum hemmenden Gas gefüllt ist.

Um den Spüleffekt beim Spülen des Behandlungsraum mit Gas zu verbessern, ist vorgesehen, daß der Gaseinlaß mehrere Gaseinlaßöffnungen umfaßt, die im Umfangsabschnitt der Haube umfangsmäßig mit Abstand zu einander angeordnet sind.

Eine zweckmäßige Ausgestaltung der Erfindung zeichnet sich dadurch aus, daß als Gasauslaß eine Auslaßventilanordnung vorgesehen ist, die zumindest eine Auslaßöffnung aufweist, an die sich ein Überdruckventil anschließt, wobei vorteilhafterweise der Gasauslaß mehrere Gasauslaßöffnungen umfaßt, die im Umfangsabschnitt der Haube umfangsmäßig mit Abstand zu einander angeordnet sind.

Um einen möglichst raschen Gasaustausch im Behandlungsraum unter der Haube zu gewährleisten, ist es zweckmäßig, wenn der Gasauslaß dem Gaseinlaß bezüglich des vom ringförmigen Befestigungsabschnitt umschlossenen offenen Bereichs im wesentlichen diametral gegenüber liegt.

Dem Gasauslaß kann auch ein Filter nachgeschaltet sein, um das austretende Gas filtern zu können.

Um eine zuverlässige und bequeme Anwendung der Wundabdeckung zu ermöglichen, ist es vorteilhaft, wenn der Deckenabschnitt der Haube relativ steif und im wesentlichen eben ist, während ihr Umfangsabschnitt relativ flexibel ist.

Durch den Einsatz eines durchsichtigen Deckenabschnitts der Haube wird es ermöglicht, die Heilung der Wunde zu beobachten, ohne daß die Wundabdeckung ständig erneuert werden muß.

Durch eine faltenbalgartige Ausbildung des Umfangsabschnitts wird ein geringes Transport- und Lagervolumen für die Wundabdeckung sichergestellt, ohne daß dadurch das Volumen des von der Wundabdeckung gebildeten Behandlungsraums begrenzt wird.

Bei einer anderen Ausgestaltung der Erfindung ist vorgesehen, daß dem relativ flexiblen Umfangsabschnitt ein Stützring zugeordnet ist, der den im wesentlichen ebenen Deckenabschnitt in einem Abstand zu der durch den Befestigungsabschnitt festgelegten Fläche hält.

Obwohl es grundsätzlich möglich ist, die Wundabdeckung mit ihrem Befestigungsabschnitt mittels im medizinischen Bereich üblichen Klebebändern zu befestigen, ist es zweckmäßig, wenn der Befestigungsabschnitt auf seiner von der Haube abgewandten Hauptoberfläche mit einem Klebstoff beschichtet ist, der mit einer abziehbaren Schutzschicht abgedeckt ist.

Die vorliegende Erfindung stellt also eine neue Wundabdeckung bereit, die mit ihrem Befestigungsabschnitt so auf eine Hautfläche befestigt wird, daß der vom Befestigungsabschnitt umgebende offene Bereich über einer Wunde liegt und diese von der Haube der erfindungsgemäßen Wundabdeckung berührungslos abgedeckt wird. Nach dem Anbringen der Wundabdeckung wird dann der Raum zwischen der Wunde und der Haube zunächst bei geöffnetem Gasein- und -auslaß mit einem sterilen Gas gespült um dann bei abgesperrtem Gasauslaß über den Gaseinlaß mit dem sterilen Gas befüllt zu werden, so daß sich die Wunde in einer im wesentlichen keimfreien Umgebung befindet.

Dabei ist es besonders vorteilhaft, wenn zum Spülen und Befüllen des Raums zwischen der Wunde und der Haube, also des Behandlungsraums über der Wunde ein keimtötendes Gas, z.B. Ozon, oder ein keimwachstumhemmendes Gas, z.B. trockener Stickstoff, verwendet wird.

Die Erfindung wird im folgenden beispielsweise anhand der Zeichnung näher erläutert, es zeigen:
**Figur 1** einen schematischen Schnitt durch eine Wundabdeckung nach einer ersten Ausgestaltung der Erfindung,
**Figur 2** einen schematischen Schnitt durch eine Wundabdeckung gemäß einer anderen Ausgestaltung der Erfindung,
**Figur 3** eine vereinfachte schematische Draufsicht auf eine erfindungsgemäße Wundabdeckung und
**Figur 4** eine vereinfachte schematische Draufsicht auf eine erfindungsgemäße Wundabdeckung mit angeschlossener Gasquelle zur Veranschaulichung einer anderen Ausbildung der Gasein- und -auslässe.

In den verschiedenen Figuren der Zeichnung sind einander entsprechende Teile mit gleichen Bezugszeichen versehen.

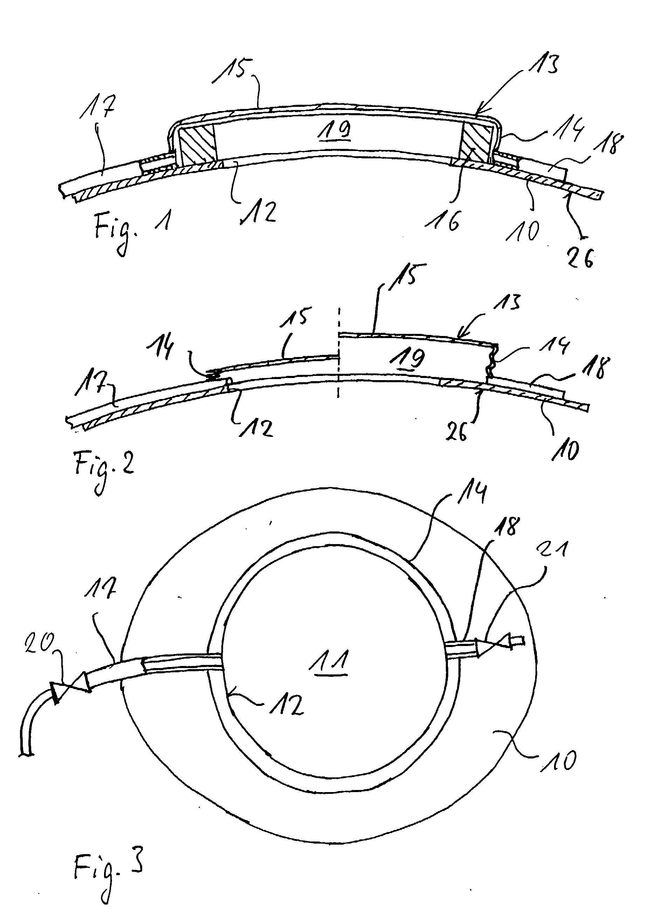

Wie in Figur 1 und 3 dargestellt ist, weist eine erfindungsgemäße Wundabdeckung einen Befestigungsabschnitt 10 mit einem offenen Bereich 11 der von einem inneren Rand 12 des Befestigungsabschnitts 10 umgeben ist. Eine Haube 13 ist mit einem Umfangsabschnitt 14 mit Abstand zum inneren Rand 12 des Befestigungsabschnitts 10 dicht angebracht, so daß ein Deckenabschnitt 15 über dem offenen Bereich 11 des Befestigungsabschnitts 10 liegt. Der Deckenabschnitt ist dabei plan oder leicht gewölbt, also im wesentlichen eben.

Um einen Abstand zwischen dem Deckenabschnitt 15 der Haube 13 und dem Befestigungsabschnitt 10 bzw. einer durch den Befestigungsabschnitt 10 gegebenen Fläche zu gewährleisten, ist ein Stützring 16 auf dem ringförmigen Abschnitt des Befestigungsabschnitts 10 zwischen dem Umfangsabschnitt 14 der Haube 13 und dem inneren Rand 12 des Befestigungsabschnitts angeordnet.

Eine Gaseinlaßleitung 17 ist mit ihrem Gasauslaßende gasdicht durch den Umfangsabschnitt 14 der Haube 13 hindurch geführt und bildet so einen Gaseinlaß. Je nach Ausbildung des Stützrings 16 aus mehr oder weniger luft- oder gasdurchlässigem Material, kann vorgesehen sein, daß sich die Gaseinlaßleitung 17 in nicht näher dargestellter Weise unter dem Stützring 16 hindurch bis zum inneren Rand 12 des Befestigungsabschnitts 10 hin erstreckt, wie dies in Figur 3 angedeutet ist.

Eine Gasauslaßleitung 18 ist mit ihrem Gaseinlaßende gasdicht durch den Umfangsabschnitt 14 der Haube 13 hindurchgeführt, um Luft und/oder Gas aus einem zwischen der Haube 13 und einer Wunde liegenden Behandlungsraum 19 ablassen zu können. Wie die Gaseinlaßleitung 17 kann dabei auch die Gasauslaßleitung 18 mit ihrem einlaßseitigen Ende unter dem Stützring 16 hindurch geführt sein.

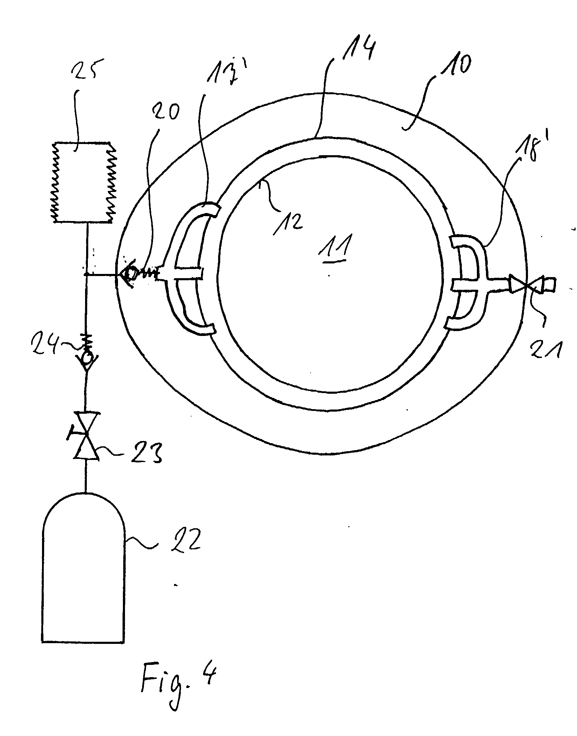

Wie in Figur 3, in der der Stützring 16 nicht dargestellt ist, gezeigt ist, sind in der Gaseinlaßleitung 17 und in der Gasauslaßleitung 18 Absperrventile 20 bzw. 21 angeordnet. Als Absperrventil 20 in der Gaseinlaßleitung 17 kann dabei, wie in Figur 4 gezeigt ist, ein federbeaufschlagtes Rückschlagventil eingesetzt werden. Es ist jedoch auch möglich, ein handbetätigtes Ventil vorzusehen.

Als Absperrventil 21 in der Gasauslaßleitung 18 kann ebenfalls ein handbetätigtes Absperrventil eingesetzt werden. Besonders vorteilhaft ist es jedoch, wenn anstelle eines handbetätigten Absperrventils oder parallel dazu ein Druckbegrenzungsventil als einziges oder zusätzliches Absperrventil eingesetzt wird, da sich mit einem derartigen Druckbegrenzungsventil sicher stellen läßt, daß der Druck innerhalb des Behandlungsraums 19 einen vorgegebenen die Wundheilung nicht beeinträchtigenden Überdruck nicht übersteigt. Dem Absperrventil 21 kann ein Filter 2la nachgeschaltet sein, um aus dem Behandlungsraum 19 ausströmendes Gas zu filtern und um dadurch zu verhindern, daß Schadstoffe in die Umgebung gelangen.

An Stelle der in Figur 3 dargestellten Gaseinlaß- und -auslaßleitungen 17, 18 können auch Leitungsanordnungen 17', 18' als Gasein- und -auslässe eingesetzt werden, die sich flußdeltaartig verzweigen, um über den Umfang des Umfangsabschnitts 14 verteilte Gaseinlaß- und -auslaßbereiche zu bilden. Es sind auch sich trichterförmig erweiternde Leitungsanordnungen oder Umfangskanäle mit einer Vielzahl von Öffnungen hierfür denkbar.

Wie in Figur 4 angedeutet ist, wird als Gasquelle zweckmäßigerweise eine Druckgasflasche 22 mit Handventil 23 eingesetzt, die über ein Rückschlagventil 24, das vorzugsweise federbeaufschlagt ist, mit einer als Dosiereinrichtung dienenden Blasebalgpumpe 25 verbunden ist.

Wie in Figur 2 dargestellt ist, kann die Haube 13 mit einem faltenbalgartig ausgebildeten Umfangsabschnitt 14 versehen sein, so daß für Transport und Lagerung der Deckenabschnitt 15 wie in der linken Hälfte in Figur 2 gezeigt dicht am Befestigungsabschnitt 10 angeordnet ist. Nach Anbringen der Wundabdeckung über einer Wunde wird der Raum zwischen der Haube 13 und der Wunde mit Gas gefüllt, so daß der Deckenabschnitt 15 von einem im Behandlungsraum 19 gebildeten Gaspolster getragen und in Abstand von der Wunde gehalten wird.

Um die erfindungsgemäße Wundabdeckung in besonders einfacher Weise über einer Wunde anordnen zu können, ist die von der Haube 13 abgewandte Hauptoberfläche des Befestigungsabschnitts 10 mit einer Klebstoffbeschichtung 26 versehen, die während des Transports mit einer abziehbaren Schicht, beispielsweise einer abziehbaren Folie oder einem abziehbaren Film abgedeckt ist.

Im folgenden wird die Handhabung der erfindungsgemäßen Wundabdeckung im einzelnen näher erläutert.

Nachdem der Befestigungsabschnitt mit der Klebstoffbeschichtung auf einer eine Wunde umgebenden Hautfläche dicht aufgebracht ist, werden zunächst die Absperrventile 21, 20 in der Gasauslaßleitung 18, 18' bzw. in der Gaseinlaßleitung 17, 17' geöffnet und ein steriles Gas wird zum Spülen des Behandlungsraum 19 in diesen eingeleitet. Wird dabei zum Einleiten von Gas eine Gasquelle verwendet, wie sie in Figur 4 angedeutet ist, so wird hierzu das Handventil 23 der Druckgasflasche 22, das als Druckreduzierventil ausgebildet sein kann, geöffnet, so daß unter Druck stehendes Gas durch das Rückschlagventil 24 in die Blasebalgpumpe 25 einströmen kann. Durch eine geeignete Ausbildung der Federkräfte der federbeaufschlagten Rückschlagventile 24 und 20 kann dabei erreicht werden, daß das Rückschlagventil 24 bei entsprechend gefüllter Blasebalgpumpe 25 bereits schließt, bevor das als Rückschlagventil 20 ausgebildete Absperrventil 20 öffnet. In diesem Fall wirkt die Blasebalgpumpe 25 als Dosiereinrichtung.

Sobald die Blasebalgpumpe 25 gefüllt ist, wird steriles Gas aus dieser über das Absperrventil 20 in den Behandlungsraum 19 eingeleitet. Die Gaseinleitung in den Behandlungsraum wird dabei bei geöffnetem Absperrventil 21 in der Gasauslaßleitung 18 bzw. 18' so lange fortgesetzt, bis der Behandlungsraum 19 hinreichend gespült ist. Daraufhin wird das Absperrventil 21 geschlossen und der Behandlungsraum 19 wird mit einer entsprechenden Menge sterilen Gases gefüllt. Die Gasquelle kann dann zweckmäßigerweise von der Gaseinlaßleitung 17, 17' getrennt werden.

Zum Begasen des Behandlungsraums 19 werden zweckmäßigerweise sterile, keimtötende und/oder keimwachstumhemmende Gase, wie beispielsweise Ozon oder trockener Stickstoff eingesetzt.

Das Spülen und Befüllen des Behandlungsraums 19 mit Gas kann dann je nach Fortschritt der Wundheilung in geeigneten Abständen wiederholt werden.

An Stelle der beschriebenen Blasebalgpumpe 25 ist es auch denkbar, eine Druckflasche oder eine andere Druckgasquelle über ein Drucksteuerventil direkt an das Absperrventil 20 der Gaseinlaßleitung 17 anzuschließen. Obwohl es grundsätzlich möglich ist, die Haube 13 aus undurchsichtigem Material herzustellen, ist es in vielen Fällen zweckmäßig, wenn zumindest der Deckenabschnitt 15 durchsichtig ist, um den Fortschritt der Wundheilung ohne Entfernen der Wundabdeckung kontrollieren zu können.

## Patentansprüche

1. Wundabdeckung mit
- einem ringförmigen Befestigungsabschnitt (10), der einen offenen Bereich (11) umschließt, und
- einer den offenen Bereich (11) überdeckenden Haube (13), die mit ihrem Umfangsabschnitt (14) luftdicht mit dem Befestigungsabschnitt (10) verbunden ist und deren über dem offenen Bereich (11) liegender Deckenabschnitt (15) einen Abstand zu einer durch den Befestigungsabsehnitt (11) festgelegten Fläche aufweist,
dadurch gekennzeichnet,
- daß sich durch die Haube (13) ein absperrbarer Gaseinlaß (17, 17', 20) zum Befüllen der Haube (13) mit einem Gas sowie ein absperrbarer Gasauslaß (18, 18', 21) zum Ablassen des Gases hindurch erstrecken.

2. Wundabdeckung nach Anspruch 1, dadurch gekennzeichnet, daß der Gaseinlaß von einer Gasleitung (17, 17') gebildet wird, die sich durch den Umfangsabschnitt (14) der Haube (13) gasdicht hindurch erstreckt, in der ein Absperrventil (20) angeordnet ist und an die in Strömungsrichtung vor dem Absperrventil (20) eine Gasquelle anschließbar ist.

3. Wundabdeckung nach Anspruch 2, dadurch gekennzeichnet, daß das Absperrventil (20) von einem Rückschlagventil gebildet wird.

4. Wundabdeckung nach Anspruch 2 oder 3, dadurch gekennzeichnet, daß die Gasquelle einen Gasvorratsbehälter (22) und eine als Dosiereinrichtung dienende Blasebalgpumpe (25) umfaßt.

5. Wundabdeckung nach Anspruch 2, 3 oder 4, dadurch gekennzeichnet, daß die Gasquelle als Gasvorratsbehälter einen Druckbehälter (22) aufweist, der mit einem unter Druck stehenden sterilen, keimtötenden und/oder das Keimwachstum hemmenden Gas gefüllt ist.

6. Wundabdeckung nach einem der vorstehenden Ansprüche, dadurch gekennzeichnet, daß der Gaseinlaß (17') mehrere Gaseinlaßöffnungen umfaßt, die im Umfangsabschnitt (14) der Haube (13) umfangsmäßig mit Abstand zu einander angeordnet sind.

7. Wundabdeckung nach einem der vorstehenden Ansprüche, dadurch gekennzeichnet, daß als Gasauslaß (18, 18') zumindest eine Auslaßöffnung vorgesehen, an die sich ein Überdruckventil (21) anschließt.

8. Wundabdeckung nach Anspruch 7, dadurch gekennzeichnet, daß der Gasauslaß (18') mehrere Gasauslaßöffnungen umfaßt, die im Umfangsabschnitt (14) der Haube (13) umfangsmäßig mit Abstand zu einander angeordnet sind.

9. Wundabdeckung nach Anspruch 7 oder 8, dadurch gekennzeichnet, daß der Gasauslaß (18, 18') dem Gaseinlaß (17, 17') bezüglich des vom ringförmigen Befestigungsabschnitt (10) umschlossenen offenen Bereichs (11) im wesentlichen diametral gegenüber liegt.

10. Wundabdeckung nach einem der vorstehenden Ansprüche, dadurch gekennzeichnet, daß der Deckenabschnitt (15) der Haube (13) relativ steif und im wesentlichen eben ist, während ihr Umfangsabschnitt (14) relativ flexibel ist.

11. Wundabdeckung nach einem der vorstehenden Ansprüche, dadurch gekennzeichnet, daß der Deckenabschnitt (15) der Haube (13) durchsichtig ist.

12. Wundabdeckung nach einem der vorstehenden Ansprüche, dadurch gekennzeichnet, daß der Umfangsabschnitt (14) als faltenbalgartig ausgebildet ist.

13. Wundabdeckung nach einem der vorstehenden Ansprüche, dadurch gekennzeichnet, daß dem relativ flexiblen Umfangsabschnitt (14) ein Stützring (16) zugeordnet ist, der den im wesentlichen ebenen Deckenabschnitt (15) in einem Abstand zu der durch den Befestigungsabschnitt (10) festgelegten Fläche hält.

14. Wundabdeckung nach einem der vorstehenden Ansprüche, dadurch gekennzeichnet, daß der Befestigungsabschnitt (10) auf seiner von der Haube (13) abgewandten Hauptoberfläche mit einem Klebstoff (26) beschichtet ist, der mit einer abziehbaren Schutzschicht abgedeckt ist.

15. Wundabdeckung nach einem der Ansprüche 1 bis 14, dadurch gekennzeichnet, daß dem Gasauslaß (18, 18', 21) ein Filter (21a) nachgeschaltet ist.

16. Verfahren zum Anbringen einer Wundabdeckung nach einem der vorstehenden Ansprüche, bei dem die Wundabdeckung mit ihrem Befestigungsabschnitt (10) so auf einer Hautfläche befestigt wird, daß der offene Bereich (11) über einer Wunde liegt und diese von der Haube (13) berührungslos abgedeckt ist, dadurch gekennzeichnet, daß der Raum (19) zwischen der Wunde und der Haube (13) zunächst bei geöffnetem Gasein- und -auslaß (17, 17', 18, 18') mit einem sterilen Gas gespült wird, um dann bei abgesperrtem Gasauslaß (18, 18') über den Gaseinlaß (17, 17') mit dem sterilen Gas befüllt zu werden, so daß die Wunde sich in einer im wesentlichen keimfreien Umgebung befindet.

17. Verfahren nach Anspruch 16, dadurch gekennzeichnet, daß der Raum (19) zwischen der Wunde und der Haube (13) mit einem keimtötenden Gas, insbesondere mit Ozon befüllt wird.

18. Verfahren nach Anspruch 16, dadurch gekennzeichnet, daß der Raum (19) zwischen der Wunde und der Haube (13) mit einem das Keimwachstum hemmenden Gas, insbesondere mit Stickstoff befüllt wird.
